# EUROPEAN PATENT APPLICATION

(11) **EP 1 683 497 A1**
(43) Date of publication of application: **26.07.2006**
(21) Application number: 06001061.8
(22) Date of filing: 18.01.2006
(51) Int. Cl.: A61C 3/00

(54) **Dental plaque collecting tool**

(30) Priority: 20.01.2005 JP 2005013041
(71) Applicant: GC Corporation, Tokyo 174-8585 (JP)
(72) Inventor: Matsumoto, Yuko, Tokyo (JP); Yoshii, Eiichi, Tokyo (JP)
(74) Representative: Müller-Boré & Partner Patentanwälte

(57) **Abstract**

To provide a new dental plaque collecting tool used for collecting a dental plaque from a tooth face and evaluating a carious activity in an oral cavity by a color change of the collected dental plaque, the dental plaque collecting tool is structured such that a neck part (2) is formed at one end of a handle part (1) having a pillar shape, the neck part (2) has an upper edge having a curved face (2a), the neck part (2) has a shape where the thickness between edges on the both sides becomes gradually thin as going to the dental plaque collecting part (3), a dental plaque collecting part (3) is formed through the neck part (2), and the dental plaque collecting part (3) comprises an upper edge having an extending plane (3a) of a tangent angle at a curved face (2a) terminal end of the neck part (2) and a lower edge, which is connected with the top end of the neck part (2) passing through a lower edge face (3b) having an acute angle from the top end of the extending plane (3a) and a convex part (3c).

## Description

The present invention relates to a dental plaque collecting tool used for collecting dental plaque from a tooth surface and evaluating carious activity in an oral cavity by a change of a color of the collected dental plaque.

A carious activity evaluation in a dentistry has an objective for predicting and judging carious active performance, that is, whether the caries of a tooth is advanced or not, or whether the caries may occur in the future by the carious activity or not. Thus, the carious activity evaluation has an important meaning on oral hygiene.

It is generally considered that the caries of the tooth is caused and advanced by a decalcification phenomenon, where hydrocarbons are metabolized by cariogenic bacteria existing in the dental plaque adhered to the tooth, to thereby generate an acid, and a calcium ion and a phosphoric acid ion in tooth are eluted with the acid. It is also considered that the discharge amount of the acid from the dental plaque for causing and advancing the caries of the tooth depends on the number of the cariogenic bacteria in the dental plaque. Thus, as one of methods for evaluating the carious activity, a measuring test of the number of mutans streptococci, a measuring test of the number of lactic acid bacilli or the like has been carried out for measuring the number of the cariogenic bacteria in the dental plaque.

However, in order to evaluate the carious activity by these methods, there are problems that an expensive culture equipment, an advanced operation technique, and a long time culturing are needed. Thus, it is not realistic for the general dentist or the like to evaluate the carious activity for a patient by using these methods, and thus the carious activity evaluation is not actually carried out, although it has an important meaning for a patient.

Then, as a method for evaluating the carious activity without the expensive culture equipment, the advanced operation technique and the long time culturing, a method measuring the discharge amount of the acid was developed. More particularly, for example, it is a method comprising the steps of preparing a chemical liquid for the carious activity evaluation containing sugar and a pH indicator, putting the dental plaque taken from the tooth surface into such the chemical liquid, and thereby evaluating the discharge amount of the acid from the dental plaque by the color change shown by the pH indicator in the chemical liquid, where the sugar is a source of carbons promoting the acid production of the cariogenic bacteria, and the pH indicator is for observing the change of the pH value by the acid generation by the cariogenic bacteria (for example, referring to Japanese Patent Application Laid Open No. 50-1589, 54-47700, 56-96700, 56-120623, Japanese Patent Publication No. 57-13824, Japanese Patent Application Laid Open No. 59-99354).

As for such a method for measuring the discharge amount of the acid produced from the dental plaque, since the activity performance of the caries can be evaluated only by putting the dental plaque into the chemical liquid for evaluating the carious activity, the simplicity is enhanced as compared with the conventional method for measuring the number of the cariogenic bacteria in the dental plaque. However, since it is necessary to put the collected dental plaque into an aqueous solution, the dental plaque is dispersed in the chemical liquid, to thereby dilute the acid produced by the cariogenic bacteria in the dental plaque. As the result of this, there is a problem that the time required for judging is too long, that is, it takes 30 minutes to 48 hours after the dental plaque is put into the chemical liquid until the color of the pH indicator in the chemical liquid for evaluating the carious activity is changed.

Further, when the dental plaque is collected for putting into the chemical liquid, there is no special tool for collecting the dental plaque which exists on a tooth surface, between a tooth and a gum, and between teeth. Thus, a general dental tool having a sharp tip such as a scaler or the like must be substituted and only few amounts of the dental plaque capable of taking by such a sharp tip can be collected by such a dental tool. Thus, in order to obtain the specified amount of the dental plaque necessary for the carious activity evaluation, the work for collecting the dental plaque by such the dental tool and putting it into the chemical liquid must be repeated several times, so that there is a problem that the workability is remarkably bad. Further, since it cannot be easily decided whether the specified amount of the dental plaque necessary for the carious activity evaluation is obtained or not, the amounts of the dental plaque may be mistaken, so that there is a problem that the accurate evaluation of the carious activity cannot be carried out. Further, as for such a dental tool, since the shape varies by the portion of a dentition or a tooth according to an object for a treatment, the suitable shape of dental tool must be chosen according to the portion of the dentition or the tooth subjected to the evaluation of the carious activity. Thus, there are problems that many kinds of dental tools must be prepared, and it takes the time and effort for choosing it.

The present invention is to solve the above-mentioned problems, and an objective of the present invention is to provide a new dental plaque collecting tool easily used for collecting the dental plaque from the tooth surface and evaluating the carious activity in the oral cavity by the color change of the collected dental plaque, whereby anybody can easily evaluate the carious activity for a short time.

The earnest work was carried out in order to solve the above-mentioned problems and, as a result of this, the followings were found to complete the present invention. When a dental plaque collecting tool has a dental plaque collecting part which is provided at one end of a handle part having a pillar shape through a neck part having a shape capable of easily working without being interfered by another tooth even when collecting a dental plaque on the surface of a back tooth on the tongue side, which has a shape of being capable to easily collect not only the dental plaque adhered on the surface of the tooth but also the dental plaque existing between the tooth and the gum or between the teeth, and not damaging the inside of the oral cavity, and which also has a shape of being capable of accumulating the specified amount of the dental plaque necessary for the carious activity evaluation, then, it is possible to easily collect the dental plaque of various portions of the tooth even when the tooth is any one of the teeth in the dentition, and to grasp the dental plaque accumulated on the dental plaque collecting part in the specified amount necessary for the carious activity evaluation by visual check. Thus, the amount of the dental plaque are measured without mistaking, and the specified amount of the dental plaque necessary for the carious activity evaluation can be easily collected in a short time, so that the workability can be remarkably enhanced.

That is, the present invention relates to the dental plaque collecting tool comprising the handle part having a pillar shape, and the dental plaque collecting part formed at the one end of the handle part through the neck part. The dental collecting tool is used for collecting the dental plaque from the tooth surface, directly dipping the dental plaque in the chemical liquid for evaluating the carious activity, and evaluating the carious activity in the oral cavity with the color change of the collected dental plaque.

The neck part comprises an upper edge having a curved face, which has a portion positioned on the lower side of an upper edge extending line of the handle part and a continuing portion positioned on the upper side of the upper edge extending line, and a lower edge positioned on the upper side of a lower edge extending line of the handle part, and has a shape where a width between edges on the both sides becomes gradually thin toward the dental plaque collecting part.

The dental plaque collecting part comprises an upper edge being an extending plane from a curved face of the neck part and a lower edge being connected with a top end of the neck part from the extending plane through a lower edge face having an acute angle and a convex part. The dental plaque collecting part is formed in a plane shape having a width between the both side edges of 0.2 to 0.7 mm and an area of the 2 to 10 mm². Further, an angle formed by the extending plane of the upper edge and the upper edge of the handle part is 90 to 115 degrees.

Further, the followings were also found out. When the length of the handle part is 70 to 120 mm, the thickness of the handle part is 2 to 5 mm, and a cross-sectional shape of the handle part is a regular polygonal shape, then, since an operator can easily handle the handle part, the workability is increased, so that it is preferable.

Further, the followings were also found out. When a constriction part is provided at the lower edge of the neck part on the handle part side, the dental plaque collecting tool according to the present invention can be easily placed on aplacing table having a convex shape corresponding to the constriction part, in the state where the top edge of the dental plaque collecting part is directed upwardly, so that it is preferable. Further, when an angle of the edges on the both sides of the neck part is 7 to 10 degrees, and the curved face of the neck part has an arc shape with a radius of 8 mm to 9 mm, and has the length of 12 to 18 mm, and when the neck part is formed to have a shape where the thickness between the upper and lower edges becomes gradually thin toward the dental plaque collecting part, then, the operativity when collecting the dental plaque by the dental plaque collecting part is enhanced, so that it is preferable.

Further, the followings are also found out. When the length of the upper edge of the dental plaque collecting part is 3 to 7 mm, the dental plaque is easily accumulated, so that it is preferable. Further, when the angle formed by the upper edge and the lower edge at the top end of the dental plaque collecting part is 30 to 60 degrees, the operativity when collecting the dental plaque existing between teeth is enhanced, so that it is preferable. Further, when the handle part, the neck part and the dental plaque collecting part are integrally formed with a crystalline thermoplastic resin, chemical resistance is excellent, so that it is preferable.

The dental plaque collecting tool according to the present invention has the above-mentioned constitution. Thus, it is possible to easily collect the dental plaque of various portions of a tooth even when the tooth is any one of teeth in the dentition. It is also possible to grasp the dental plaque accumulated on the dental plaque collecting part in the specified amount necessary for the carious activity evaluation by visual check. Thus, measuring the amount of the dental plaque is not mistaken, and the specified amount of the dental plaque necessary for the carious activity evaluation can be easily collected in a short time, so that the workability can be remarkably enhanced. Further, when the carious activity evaluation is carried out using the dental plaque collecting tool according to the present invention, only the following steps must be carried out. The steps comprises collecting of the specified amount of the dental plaque necessary for the carious activity evaluation from the tooth surface by the dental plaque collecting part, directly dipping the dental plaque collecting part, where the dental plaque is accumulated, in the chemical liquid for evaluating the carious activity, immediately taking out the dental plaque collecting part from the chemical liquid, and leaving it on for a specified time. Then, the carious activity in the oral cavity can be evaluated by the color change of the collected dental plaque. Thus, the carious activity can be evaluated easily in a short time as compared with the conventional method of the carious activity evaluation where the dental plaque is put into the chemical liquid for evaluating the carious activity.

That is, in the conventional method of the carious activity evaluation where the dental plaque is put into the chemical liquid for evaluating the carious activity, since it is necessary to put the collected dental plaque into the aqueous solution, the dental plaque is dispersed in the chemical liquid for carious activity evaluation to thereby dilute the acid produced by the cariogenic bacteria in the dental plaque. Further, it takes 30 minutes to 48 hours after the dental plaque is put in until the color shown by the pH indicator in the chemical liquid is changed, so that the long time is necessary for judging. On the other hand, in the method of the carious activity evaluation using the dental plaque collecting tool according to the present invention, the dental plaque collecting part, where the specified amount of the dental plaque, is accumulated, is directly dipped in the chemical liquid, and the dental plaque collecting part is immediately taken out from the chemical liquid. Thus, the dental plaque accumulated on the dental plaque collecting part, which is taken out from the chemical liquid, is impregnated with the suitable amount of chemical liquid for promoting the acid production by the cariogenic bacteria in the dental plaque, and the acid produced from the dental plaque is not diluted. Therefore, the pH indicator in the chemical liquid is sharply reacted to change the color. Thus, the evaluation can be carried out only by leaving the dental plaque collecting part, where the dental plaque impregnated with the suitable chemical liquid for evaluating the carious activity is accumulated, for a short time, without observing the color change of the pH indicator in the chemical liquid after expending long time for culturing as done in the conventional method.

Further, the length of the handle part is 70 to 120 mm, the thickness of the handle part is 2 to 5 mm, and a cross-sectional shape of the handle part has a regular polygonal shape. Then, since an operator can easily handle the handle part, the workability is enhanced, so that it is preferable. Further, when the cross-sectional shape of the handle part has the regular polygonal shape, it is possible to grasp the direction of the top end of the dental plaque collecting part by the feeling of fingers when holding the handle part, so that it is preferable.

Further, when the constriction part is provided at the lower edge of the neck part on the handle part side, the dental plaque collecting tool according to the present invention can be easily place d on the placing table having a placing part, where a convex shape corresponding to the constriction part is formed, in the state where the top edge of the dental plaque collecting part is directed upwardly. Thus, when leaving the dental plaque collecting part where the dental plaque impregnated with the suitable amount of the chemical liquid is accumulated, the dental plaque collecting tool according to the present invention can be stably let alone without holding the handle part, so that it is preferable. Further, when the angle of the edges on the both sides of the neck part is 7 to 10 degrees, the balance between the operativity in the oral cavity and the strength of the neck part at the time of collecting the dental plaque is good, so that it is preferable. Further, when the curved face of the neck part has an arc shape of 8 mm to 9 mm radius and 12 to 18 mm length, the neck part has a shape closing to the shape along the tooth surface, so that the operation can be efficiency carried out at even a place which is interfered by another tooth, such as the tongue side of a back tooth or the like, so that it is preferable. Further, when the neck part is formed to have a shape where the thickness between the upper and lower edges becomes gradually thin toward the dental plaque collectingpart, a narrow place, such as a place between a tooth and a gum, a place between teeth or the like, can be easily operated, so that it is preferable.

Further, when the length of the upper edge of the dental plaque collecting part is 3 to 7 mm, the dental plaque is easily accumulated, and the dental plaque existing between a tooth and a gum can be easily collected, so that it is preferable. Further, when the angle formed by the upper edge and the lower edge of the top end of the dental plaque collecting part is 30 to 60 degrees, the operativity when collecting the dental plaque on the tongue side of a back tooth is enhanced, and, such a place as between teeth, on the occlusal surface of a molar or the like can be easily operated, so that it is preferable. Further, when handle part, the neck part and the dental plaque collecting part are integrally formed with the crystalline thermoplastic resin, the chemical resistance is excellent, so that it is preferable.

Figure 1 is an explanatory side view of one example of a dental plaque collecting tool according to the present invention.

Figure 2 is an explanatory plan view of Figure 1.

Figure 3 is an explanatory perspective view of Figure 1.

Figure 4 is an explanatory perspective view illustrating a state where the dental plaque collecting tool illustrated in Figure 1 is placed on a placing table.

Hereinafter, the dental plaque collecting tool according to the present invention is described concretely with drawings.

In the drawings, 1 is a handle part having a pillar shape. On the one end of this handle part 1, a dental plaque collecting part 3, which is formed through a neck part 2, is provided. The handle part 1 is held by an operator when collecting the dental plaque on the tooth face by the dental plaque collecting part 3.

The handle part 1 is not limited especially if it is an approximately linear part formed in a pillar shape. However, from an operational viewpoint, it is preferable that the length of the handle part 1 is 70 to 120 mm, and the thickness is 2 to 5 mm. Further, when the cross-sectional shape of the handle part 1 is the regular polygonal shape, it is possible to grasp the direction of the top end of the dental plaque collecting part by the feeling of fingers, so that it is preferable. Further, when the handle part 1 is placed on a placing part S of a placing table T after collecting the dental plaque, it is hardly fallen, so that it is preferable.

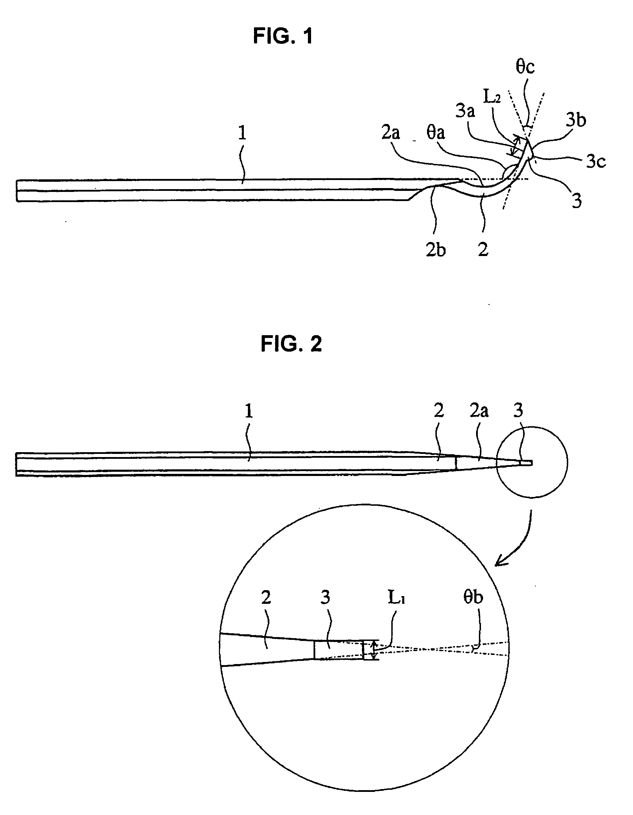

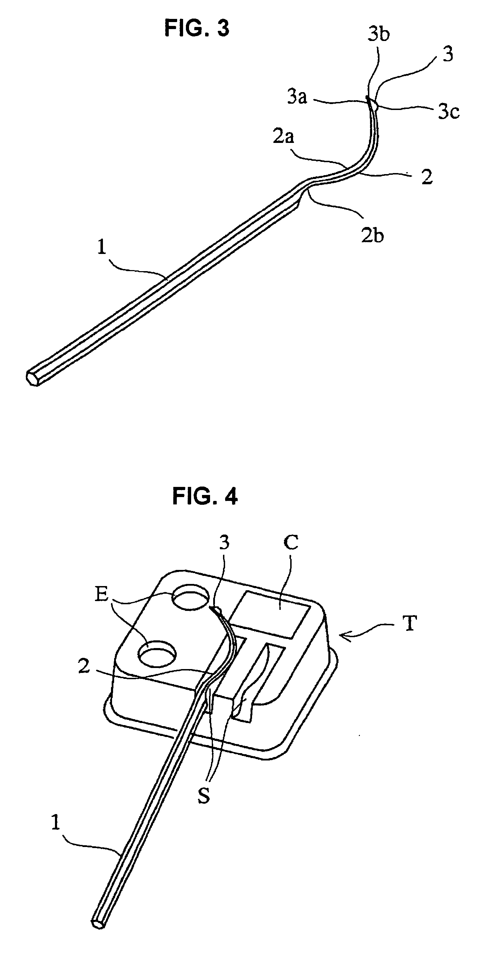

2 is a neck part for connecting one end of the handle part 1 with the dental plaque collecting part 3. As illustrated in Figures 1 and 3, the neck part 2 comprises an upper edge having a curved face 2a, which has a portion positioned on the lower side of the upper edge extending line of the handle part 1, and a continuing portion positioned on the upper side of the upper edge extending line, and a lower edge positioned on the upper side of a lower edge extending line of the handle part 1, and has a shape where the width between edges on the both sides becomes gradually thin toward the dental plaque collecting part 3. Thus, the neck part 2 has a shape capable of easily operating even on the tongue side of the back tooth without being interfered by another tooth.

As illustrated in Figures 1 and 3, the upper edge of the neck part 2 has the curved face 2a, which has the portion positioned on the lower side of the upper edge extending line of the handle part 1, and the continued portion positioned on the upper side of the upper edge extending line. Thus, the curved face 2a has the shape so as to avoid another tooth when collecting the dental plaque at a place where the operation is interfered by the other tooth, for example, at a place on the tongue side of a back tooth or the like. Therefore, the neck part 2 can enhance the operativity in such , a place. More particularly, when the curved face 2a has the arc shape with the radius of 8 mm to 9 mm and the length of 12 to 18 mm, the shape of the neck part 2 becomes close to the shape along the tooth surface of the tooth. Thus, the operation is carried out more efficiently, so that it is preferable.

Further, the lower edge of the neck part 2 must be positioned on the upper side of the lower edge extending line of the handle part 1 as illustrated in Figure 1. The reason of this is that, if the lower edge of the neck part 2 is formed to be positioned on the lower side of the lower edge extending line of the handle part 1, the lower edge of the neck part 2 is contacted with the lip face at the time of the operation, to thereby deteriorate the operativity.

Further, the neck part 2 must be formed to have the shape, where the width between edges on the both sides becomes thin gradually toward the dental plaque collectingpart 3, as illustrated in Figure 2. The reason of this is that the processing becomes easy to thereby enhance the productivity. Further, when an angle θb of the edges on the both sides of the neck part 2 is 7 to 10 degrees, the balance between the operativity in the oral cavity and the strength of the neck part at a time of collecting the dental plaque by the dental plaque collecting part is good, so that it is preferable. When the angle θb of the edges on the both sides of the neck part 2 is less than 7 degrees, the strength of the whole neck part may be insufficient. When the angle θb of the edges on the both sides of the neck part 2 is more than 10 degrees, the whole neck part becomes too thick, and thus the operativity in the oral cavity may be deteriorated.

Furthermore, when the neck part 2 is formed to have the shape where the thickness between the upper and lower edges becomes gradually thin toward the dental plaque collecting part 3 as illustrated in Figures 1 and 3, the operation can be easily carried out even at a narrow place such as the place between a tooth and a gum, a place between teeth or the like, so that it is preferable. Further, when the constriction part 2b is provided at the lower edge of the neck part 2 on the handle part 1 side as illustrated in Figures 1 and 3, the dental plaque collecting tool according to the present invention can be easily placed on the placing table T having the placing part S where the convex shape corresponding to the constriction part is formed, in the state where the top edge of the dental plaque collecting part 3 is directed upwardly, as illustrated in Figure 4. Thus, when the dental plaque collecting part 3 having the accumulated dental plaque suitably impregnated with the chemical liquid for evaluating the carious activity is left alone, the dental plaque collecting tool according to the present invention can be stably left alone without holding the handle part 1, so that it is preferable.

3 is a dental plaque collecting part formed at one end of the handle part 1 through the neck part 2. As illustrated in Figures 1 and 3, the upper edge of the dental plaque collecting part 3 is an extending plane 3a from a curved face 2a of the neck part 2, and a lower edge is connected with the top end of the neck part 2 from the extending plane 3a through a lower edge face 3b having an acute angle and a convex part 3c. Further, as illustrated in Figure 2, the dental plaque collecting part 3 is formed in a plane shape having a width L₁ between the both side edges of 0.2 to 0.7 mm and an area of 2 to 10 mm². Further, an angle θa formed by the extending plane 3a of the upper edge and the upper edge of the handle part 1 is 90 to 115 degrees. Thus, the dental plaque collection part 3 can collect the dental plaque existing between a tooth and a gum or between teeth, in addition to the dental plaque adhered on a tooth surface of a tooth, and accumulate the specified amount of the dental plaque needed for the carious activity evaluation.

Since the upper edge of the dental plaque collection part 3 is the extending plane 3a from the curved face 2a of the neck part 2 as illustrated in Figures 1 and 3, the dental plaque adhered on a tooth surface can be collected by chipping off and removing, so that the collecting efficiency of the dental plaque is good. Further, when the length L₂ of the upper edge of the dental plaque collecting part 3 is 3 to 7 mm, the dental plaque can be easily accumulated, so that it is preferable. Further, the dental plaque existing between a tooth and a gum can be easily collected, so that it is preferable.

Since the lower edge of the dental plaque collecting part 3 is connected with the top end of the neck part 2 from the extending plane 3a through the lower edge face 3b having the acute angle and the convex part 3c as shown in Figures 1 and 3, thus, the dental plaque existing between a tooth and a gum or between teeth can be easily collected by the top end having the acute angle, the extending plane 3a and the lower edge face 3b, and it can be prevented to damage the inside of the oral cavity by the convex part 3c continuing from the lower edge face 3b. Further, when the angle θc of the upper edge and the lower edge of the top end of the dental plaque collecting part 3 is 30 to 60 degrees, the operativity when collecting the dental plaque existing between teeth is enhanced, so that it is preferable.

Further, the dental plaque collecting part 3 is formed in a plane shape having the width L1 between both side edges of 0.2 to 0.7 mm and an area of 2 to 10 mm². The reason of this is that the dental plaque collecting part can easily enter into the place between a tooth and a gum or between teeth, and the collected dental plaque can be accumulated. Further, the angle θa of the extending plane 3a of the upper edge and the upper edge of the handle part 1 is 90 to 115 degrees, more preferably 100 to 110 degrees. The reason of this is that the dental plaque collecting part 3 is contacted with the various portions of a tooth even when the tooth is any one of teeth in the dentition, to thereby easily collect the dental plaque. Thus, the collecting efficiency of the dental plaque is good.

The dental plaque collecting tool according to the present invention comprising the handle part 1, the neck part 2 and the dental plaque collecting part 3 may be integrally formed with the material such as metal, wood, a synthetic resin or the like, or may be formed by combining these materials. However, when the handle part 1, the neck part 2 and the dental plaque collecting part 3 are integrally formed with the crystalline thermoplastic resin, the chemical resistance and formability are excellent, so that it is preferable.

Then, the method for carrying out the carious activity evaluation using the dental plaque collecting tool according to the present invention having such the constitution is described.

First, as a preparation, the chemical liquid for evaluating the carious activity is prepared, where sugar and a pH indicator are contained therein. The sugar is the carbons source promoting the acid production by the cariogenic bacteria, and the pH indicator is for founding the change of the pH value by producing the acid by the cariogenic bacteria. Further, the container is prepared, where the container is used for directly dipping the dental plaque collecting part 3 in the chemical liquid for evaluating the carious activity in the state of a dental plaque being accumulated thereon. Then, the preparation for dropping the specified amount of the chemical liquid onto the container is carried out.

At this time, as such the container, the placing table T is used as illustrated in Figure 4, where the placing table T has a concave liquid storing part E and the placing part S. The concave liquid storing part E is for dropping and storing the specified amount of chemical liquid for evaluating the carious activity in order to directly dip the dental plaque collecting part 3. The placing part S has the shape corresponding to the constriction part 2b of the neck part 2. Then, the specified amount of the chemical liquid for evaluating the carious activity necessary to evaluate the carious activity can be easily prepared without waste. Further, when the dental plaque collecting part 3 with accumulated dental plaque suitably impregnated with the chemical liquid is left alone, the dental plaque collecting tool according to the present invention can be stably left alone without holding the handle part 1, so that it is preferable. Further, when a color chart C for judging is displayed on the placing table T, the carious activity can be easily evaluated only using such the placing table T, as a color change of the dental plaque and the carious activity evaluation corresponding to the color change of the dental plaque canbe judged at a glance after impregnating the chemical liquid and leaving for a specified time. So, it is preferable.

Then, a preparation for drying saliva existing on a tooth portion is desirably carried out by spraying air to the desired tooth portion of the dentition, which is to be subjected to the carious activity evaluation. This spraying has an objective to eliminate a risk that the carious activity evaluation cannot be correctly carried out by a cause of mixing the saliva into the dental plaque to be a sample.

After such the preparations, the operation for collecting the specified amount of the dental plaque from the tooth face of the desired portion for carrying out the carious activity evaluation of the tooth of the dentition is carried out at first using the dental plaque collecting tool according to the present invention.

This operation is carried out by the steps of holding the handle part 1 of the dental plaque collecting tool according to the present invention, moving it while contacting the dental plaque collecting part 3 with the desired portion of the tooth of the dentition, collecting the dental plaque adhered on the tooth surface, and accumulating the collected dental plaque on the dental plaque collecting part 3. Further, while visually confirming the amount of the dental plaque accumulated on the dental plaque collecting part 3, this operation is repeateduntil the amount of the dental plaque becomes the amount covering approximately the half of the dental plaque collecting part 3, which is the specified amount necessary for carrying out the carious activity evaluation.

At this time, when the desired portion of the tooth of the dentition for carrying out the carious activity evaluation is a tooth surface, the dental plaque adhered on the tooth surface may be collected by being chipped off and removed with the extending plane 3a of the upper edge of the dental plaque collecting part 3. Further, when this portion is the place between a tooth or a gum or between teeth, the dental plaque may be collected by inserting the upper end of the dental plaque collecting part 3 into the objective place, and scraping out the dental plaque.

Then, the directly dipping operation is carried out, where the dental plaque collecting part 3 is directly dipped in the chemical liquid for evaluating the carious activity in the state of the specified amount of the dental plaque necessary for evaluating the carious activity being accumulated on the dental collecting part 3.

This operation may be carried out until the chemical liquid is suitably impregnated into the dental plaque collected on the dental plaque collecting part 3, so that the chemical liquid promotes the acid production of the cariogenic bacteria in the dental plaque. Although this operation depends on factors such as components of the chemical liquid for evaluating the carious activity, the amount of the dental plaque or the like, the dipping time is about 1 to 10 seconds.

Finally, an operation for evaluating the carious activity in the oral cavity is carried out by observing the color change of the collected dental plaque, after taking out the dental plaque collecting part 3 from the chemical liquid and leaving alone for a specified time.

At this time, the dental plaque accumulated on the dental plaque collecting part, which is taking out from the chemical liquid, is suitably impregnated with the chemical liquid for promoting the acid production of the cariogenic bacteria in the dental plaque. Further, the acid produced from the dental plaque is hardly diluted. Thus, the pH indicator in the chemical liquid for evaluating the carious activity reacts sharply to change the color in a short time. Further, although a leaving time in this operation depends on factors such as components of the chemical liquid for evaluating the carious activity, the amount of the dental plaque, a room temperature or the like, it is about 3 to 10 minutes. Thus, the time for the carious activity evaluation can be remarkably shortened, and the time and effort for the carious activity evaluation can be lessened as compared with the conventional method.

## Claims

1. A dental plaque collecting tool,
wherein said tool comprises a handle part (1) having a pillar shape, and a dental plaque collecting part (3) formed at one end of the handle part (1) through a neck part (2), and is used for collecting a dental plaque from a tooth surface, directly dipping the dental plaque in a chemical liquid for evaluating a carious activity, and evaluating the carious activity in an oral cavity by a color change of the dental plaque,
wherein said neck part (2) comprises:
an upper edge having a curved face (2a) comprising a portion positioned on the lower side of an upper edge extending line of the handle part (1) and a continuing portion positioned on the upper side of the upper edge extending line of the handle part (1), and
a lower edge positioned on the upper side of a lower edge extending line of the handle part (1), and
has a shape where a width between the edges on the both sides becomes gradually thin toward the dental plaque collecting part (3), and
wherein said dental plaque collecting part (3) comprises:
an upper edge being an extending plane (3a) from a curved face (2a) of the neck part (2), and
a lower edge being connected with a top end of the neck part (2) from extending plane (3a) through a lower edge face (3b) having an acute angle and a convex part (3c), said dental plaque collecting part (3) is formed in a plane shape having a width (L₁) between the both side edges of 0.2 to 0.7 mm and an area of 2 to 10 mm², and
an angle (θa) formed by the extending plane (3a) of the upper edge of said dental plaque collecting part (3) and the upper edge of said handle part (1) is 90 to 115 degrees.

2. The dental plaque collecting tool as claimed in claim 1,
wherein the length of the handle part (1) is 70 to 120 mm, and the thickness of said handle part (1) is 2 to 5 mm.

3. The dental plaque collecting tool as claimed in claim 1 or 2,
wherein the cross-sectional shape of the handle part (1) is a regular polygonal shape.

4. The dental plaque collecting tool as claimed in any one of claim 1 to claim 3,
wherein a constriction part (2b) is provided at the lower edge of the neck part (2) on the handle part (1) side.

5. The dental plaque collecting tool as claimed in any one of claim 1 to claim 4,
wherein an angle (θb) of the extending lines of the edges on the both sides of the neck part (2) is 7 to 10 degrees.

6. The dental plaque collecting tool as claimed in any one of claim 1 to claim 5,
wherein the curved face (2a) of the neck part (2) has an arc shape having a radius of 8 mm to 9 mm, and the length of 12 to 18 mm.

7. The dental plaque collecting tool as claimed in any one of claim 1 to claim 6,
wherein the neck part (2) is formed to have a shape where the thickness between the upper and lower edges becomes gradually thin toward the dental plaque collecting part (3).

8. The dental plaque collecting tool as claimed in any one of claim 1 to claim 7,
wherein a length (L₂) of the upper edge of the dental plaque collecting part (3) is 3 to 7 mm.

9. The dental plaque collecting tool as claimed in any one of claim 1 to claim 8,
wherein an angle (θc) of the upper and lower edges of the top end of the dental plaque collecting part (3) is 30 to 60 degrees.

10. The dental plaque collecting tool as claimed in any one of claim 1 to claim 9,
wherein the handle part (1), the neck part (2) and the dental plaque collectingpart (3) are integrally formed with a crystalline thermoplastic resin.
